# EUROPÄISCHE PATENTSCHRIFT

(11) **EP 0 516 795 B1**
(45) Veröffentlichungstag und Bekanntmachung des Hinweises auf die Patenterteilung: **26.05.1999**
(21) Anmeldenummer: 92901661.6
(22) Anmeldetag: 20.12.1991
(51) Int. Cl.: A61K 31/495

(54) **ARZNEIMITTEL ENTHALTEND EINEN QUISQUALAT-REZEPTOR-ANTAGONISTEN UND EINEN DOPAMIN-AGONISTEN ZUR BEHANDLUNG DES MORBUS PARKINSON**
MEDICAMENTS CONTAINING A QUISQUALATE RECEPTOR ANTAGONIST AND A DOPAMINE AGONIST FOR THE TREATMENT OF PARKINSON'S DISEASE
MEDICAMENTS CONTENANT UN ANTAGONISTE DU RECEPTEUR DE QUISQUALATE ET UN AGONISTE DU RECEPTEUR DE DOPAMIN DESTINES AU TRAITEMENT DE LA MALADIE DE PARKINSON

(30) Priorität: 21.12.1990 DE 4041981; 26.06.1991 DE 4121483
(43) Veröffentlichungstag der Anmeldung: 09.12.1992
(73) Patentinhaber: SCHERING AKTIENGESELLSCHAFT, 13342 Berlin (DE)
(72) Erfinder: KLOCKGETHER, Thomas, Dr., D-7413 Gomaringen (DE); LÖSCHMANN, Peter-Andreas, D-1000 Berlin 19 (DE); STEPHENS, David Norman, Dr., D-1000 Berlin 33 (DE); TURSKI, Lechoslaw, Dr., D-1000 Berlin 20 (DE); WACHTEL, Helmut, Dr., D-1000 Berlin 19 (DE)
(86) Internationale Anmeldenummer: DE9101006
(87) Internationale Veröffentlichungsnummer: WO9211012

(56) Entgegenhaltungen:
- EP-A- 0 260 467
- EP-A- 0 283 959
- WO-A-90/15606
- Experimental Neurology, Band 108, Nr. 3, Juni 1990, J.W. OLNEY et al.: "Brief communication. Excitotoxicity of L-DOPA and 6-OH-DOPA: implications for Parkinson's and Huntington's diseases", Seiten 269-272, siehe das ganze Dokument
- Ann. Neurol., Band 30, Nr. 5, November 1991, T. KLOCKGETHER et al.: "The AMPA receptor antagonist NBQX has antiparkinsonian effects in monoamine-depleted rats and MPTP-treated monkeys", Seiten 717-723, siehe das ganze Dokument
- J. Neural Transm. [P-D Sect.], Band 3, 1991, P.A. LÖSCHMANN et al.: "Synergism of the AMPA-antagonist NBQX and the NMDA-antagonist CPP with L-Dopa in models of Parkinson's disease", Seiten 203-213, siehe das ganze Dokument
- Science, Band 247, Nr. 4942, 2. Februar 1990, M.J. SHEARDOWN et al.: "2,3-dihydroxy-6-nitro-7-sulfamoyl-benzo(F)quinoxaline: A neuroprotectant for cerebral ischemia", Seiten 571-574, siehe das ganze Dokument
- J.E.F. REYNOLDS: "Martindale: The extra Pharmacopoeia", 1989, Ausgabe 29, The Pharmaceutical Press, (London, GB), Seiten 1015-1020, Nr. 4541-G: "Levodopa"; Seite 1010, Nr. 4544-S: "Benserazide-Hydrochloride", siehe das ganze Dokument

## Beschreibung

Die Erfindung betrifft Mittel enthaltend (1) einen Antagonisten des Quisqualat-Rezeptor-Komplexes oder dessen physiologisch verträgliche Salze und (2) einen Dopamin-Agonisten zur Prävention und Behandlung des Morbus Parkinson.

Im zentralen Nervensystem von Säugern, einschließlich der Mensenen sind hohe konzentrationen von exzitatorischen Aminosäuren wie Glutamat und Aspartat (Fonnum, F., J. Neurochem 42: 1-11 1984) verhanden. Für die exzitatorischen Aminosäuren existieren verschiedene Rezeptoren die entsprechend ihrer spezifischen Agonisten als N-methyl-D-Aspartat (NMDA)-, Kainat (KA)- und Quisqualat (QUIS) -Rezeptor bezeichnet werden. Die Quisqualat-Rezeptoren werden nach den spezifischen Agonisten (RS)-α-Amino-3-hydroxy-5-methyl -4-isoxazolpropionat auch AMPA-Rezeptoren genannt. Die synaptische Funktion der exzitatorischen Aminosäure L-Glutamat wird vorwiegend über AMPA-Rezeptoren vermittelt.

Aus klinischenen und tierexperimenteilen Befunden gibt es Hinweise, daß es bei der Parkinson'schen Krankheit (PD) als Folge des striatalen Mangels an Dopamin zu gesteigerter glutamaterger Neurotransmission in verschiedenen Kernen der Basalganglien kommt. Das Neostriatum (NEO) stellt die Eingangsstruktur der Basalganglien dar: es erhält vom Cortex eine massive glutamaterge Projektion und von der Substantia nigre pars compacta (SNC) die dopaminerge nigrostriotale Bahn, die bei der PD degeneriert. Vom NEO gibt es direkte Bannen zu den Ausgangskernen der Basalganglien, dem internen Pallidumglied (GPi) und der Substantia nigra pars reticulata (SNR), sowie indirekte, die über das externe Pallidumglied (GPe) und der Nucleus subthalamicus (STH) verlaufen. Der STH erhält eine eigene direkte glutamaterge Innervation vom Cortex; seine zu der Ausgangskernen projizierenden Neurone benutzen ebenfalls L-Glutamat als Transmitter.

Die synaptischen Funktionen von Dopamin im NEQ sind komplex. Seine Wirkung auf die zum GPe projizierenden striatalen Neurone ist vorwiegend inhibitorisch, so daß als Folge des striatalen Dopaminmangels, wie er bei der PD vorliegt, die exzitatorischen glutamatergen Einflüsse auf diese Neurone überwiegen. Da sowohl die striatale Bahn zum GPe, als auch die von dort ausgehende zum STH projizierende Bahn inhibitorisch sind, kommt es bei der PD im STR zum Phänomen der Disinhibition mit Anstieg der tonischen Zellaktivität. Über seine glutamatergen Projektionen bewirkt der STH schließlich eine pathologisch gesteigerte neuronale Aktivität in den Ausgangskernen der Basalganglien. Untersuchungen an Tiermodellen der PD zeigen, daß es nach Gabe dopaminerger Substanzen zu einer Normalisierung der gesteigerten exzitatorischen Neurotransmission kommt, die parallel zu der "klinischen" Besserung läuft. Daß Quisqualat-Rezeptor-Antagonisten als Neuroleptika verwendet werden können, ist aus EP-A-0260467 und EP-A-0283959 bekannt.

Wir haben gefunden, daß Quisqualat-Rezeptor-Antagonisten die pathologisch gesteigerte neuronale Aktivität hemmen und die glutamaterge Transmission im NEO, STH und den Ausgangskernen der Basalganglien blockieren und daher als Arzneimittel zur Behandlung der PD verwendbar sind.

Auf Grund ihres Wirkmechanismus üben Quisqualat-Rezeptor-Antagonisten auch neuroprotective Wirkung aus, insbesondere auch gegenüber den möglichen neurotoxischen Wirkungen, die durch dopaminerge Arzneimittel ausgelost werden, die in Kombination mit QA-Rezeptor-Antagonisten verabreicht werden können. Daher können sie auch als Arzneimittel zur präventiven Behandlung der PD verwendet werden.

Erfindungsgemäß geeignet sind Verbindungen oder deren physiologisch verträgliche Salze, die hohe Affinität zu den zentralen AMPA-Rezeptoren besitzen und selektiv die synaptischen Wirkungen von Quisqualat aufheben. Solche Quisqualat-Rezeptor-Antagonisten sind beispielsweise in EP-A-374 534, EP-A-348 872, EP-A-283 959 und EP-A-377 112 und EP-A-315 959 beschrieben. Insbesondere geeignet sind Quinoxalindion-Derivate und deren tautomere Formen mit selektiver und nicht selektiver Wirkung auf AMPA-Rezeptoren.

Bevorzugte Ausführungsformen sind Chinoxalindionderivate und deren tautomere Formen der Formel I 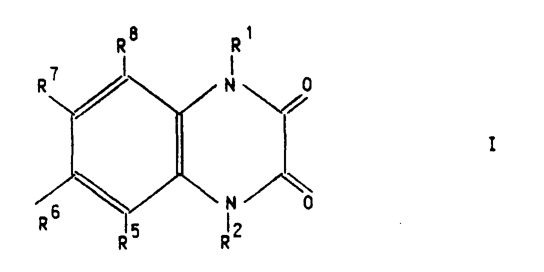 worin
R¹ und R² jeweils Wasserstoff oder einen in den zitierten Patenten genannten Substituenten darstellen und
R⁵, R⁶, R⁷ und R⁸ gleich oder verschieden sind und jeweils Wasserstoff, NO₂,NH₂, Cyano, Halogen (Fluor, Chlor, Brom oder Jod), CF₃, SO₂NR'R', SO₂R' oder OR' bedeuten und R' Wasserstoff oder C₁₋₄-Alkyl ist oder
R⁵ und R⁶ oder R⁷ und R⁸ jeweils gemeinsam einen ankondensierten Benzol-oder Hetarylring oder -(CH₂)₄- bedeuten, wobei der Benzol- oder Hetarylrest ein-bis dreifach gleich oder unterschiedlich substituiert sein kann mit NO₂` NH₂, Cyano, Halogen, CF₃, SO₂NR'R' , SO₂R' oder OR' , wobei R' die obige Bedeutung hat. Geeignete Hetarylringe sind Pyridin, Pyrazol, Thiophen, Pyrazin, Triazol, Imidazol. Geeignete Substituenten R¹ und R² sind gegebenenfalls mit Hydroxy, MH₂, Carboxy, Carbonsäureestern oder Carbonsäureamiden substituiertes C₁₋₁₂-Alkyl, C₃₋₈-Cycloalkyl, C₄₋₈-Cycloalkylalkyl, C₆₋₁₀-Aryl insbesondere Phenyl, C₇₋₁₁-Aralkyl insbesondere Benzyl, C₂₋₇-Alkanoyloxy, Hydroxy, C₁₋₆-Alkoxy, C₆₋₁₀-Aryloxy insbesondere Benzyloxy, C₃₋₈-Cycloalkyloxy und C₄₋₈-Cycloalkylalkyloxy.

Insbesondere bevorzugt sind Chinoxalin-dion und Benzochinoxalindion-Derivate, deren tautomere Formen und Salze, die gegebenenfalls ein- bis zweifach mit NO₂, Halogen, Cyano, CF₃, SO₂NR'R', SO₂R' oder OR' substituiert sind, wobei R' Wasserstoff oder C₁₋₄-Alkyl ist und R¹ und R² jeweils Wasserstoff oder einen Substituenten darstellt wie beispielsweise 6-Nitro-7-sulfamoylbenzo[f]-quinoxalin-2,3-(1H, 4H)-dion (NBQX), 6,7-Dinitroquinoxalin-2,3dion (DNQX) und 6-Cyano-7-nitroquinoxalin-2,3-dion (CNQX).

Die physiologisch verträglichen Salze leiten sich von Alkali- oder Erdalkalimetallen oder den üblichen anorganischen oder organischen Säuren wie Salzsäure, Bromwasserstoffsäure, Schwefelsäure, Phosphorsäure, Zitronensäure, Maleinsäure oder Furmarsäure ab.

Die erfindungsgemäße Wirkung wird am Beispiel von 6-Nitro-7-sulfamoylbenzo[f]-quinoxalin-2,3(1H,4H)-dion mit den folgenden Testen gezeigt

Die Versuche wurden an männlichen Wistar Ratten (Schering AG, Berlin, B.R.D.) mit einem Gewicht zwischen 220 und 240 g durchgeführt, Sie wurden unter kontrollierten Bedingungen mit freiem Zugang zu Futter und Wasser gehalten. Alle Versuche fanden zwischen 10 und 14 Uhr statt. Die Messungen begannen 5 Minuten nach der Applikation der Substanz und wurden über 60 Minuten durchgeführt.

Alle Substanzen wurden intraperitoneal (i.p.) in einem Volumen von 0,5 ml pro Kilogramm Körpergewicht verabreicht. Kontrolltiere erhielten ein entsprechendes Volumen physiologischer Kochsalzlösung. Die folgenden Substanzen wurden benutzt: Reserpin, α-Methyl-p-tyrosin, Benserazid und 6-Nitro-7-sulfamoylbenzo[f]quinoxalin-(2,3(1H,4H)-dion (NBQX). Reserpin wurde in einem Tropfen Eisessig gelöst und mit demineralisiertem Wasser verdünnt. Benserazid und α-Methyl-p-Tyrosin wurden in physiologischer Kochsalzlösung gelöst. NBQX wurde mit einer kleinen Menge 1N NaOH in Lösung gebracht und dann mit physiologischer Kochsalzlösung verdünnt. Der pH wurde auf 7,4 eingestellt.

Ein computerisiertes Digiscan-16 System (Omnitech, Columbus OH, U.S.A.) wurde zur Messung der lokomotorischen Aktivitat benutzt. Jeder Aktivitatsmesser besteht aus einem Plexiglas-Käfig (40 x 40 x 40 cm), der von 48 horizontalen und vertikalen Infrarot-Sensoren umgeben ist. Die Gesamtzahl der Lichtschrankenunterbrechungen wurde als Maß der lokomotorischen Aktivität gemessen.

Die tonische Elektromyogramm-(EMG-) Aktivität wurde vom M. gastrochemiussoleus (GS) wacher Ratten mit Hilfe Teflon-isolierter Drahtelektroden abgeleitet. Die Tiere wurden dazu in belüftete Plexiglas-Boxen gesetzt, so daß ihre Hinterbeine durch Schlitze im Boden der Boxen heraushingen. Die elektrischen Signale wurden verstärkt, gefiltert (5 Hz - 10 kHz) und gleichgerichtet. Die integrierte EMG-Aktivität wurde jeweils über Perioden von 5 Minuten gemittelt. Die Spontanaktivität der reserpinisierten Tiere vor Applikation der Substanzen wurde in 2 Segmenten gemessen und diente als Ausgangswert.

Auslösung von kontralateralen Rotationen an unilateral Substantia nigralädierten Ratten. Männliche Wistar-Ratten wurden 8 Monate vor dem Experiment durch Injektion von 16 µg 6-Hydroxydopamin (6-OHDA) in die linke Substantia nigra lädiert. Die Messung der Rotationen erfolgte in automatisierten Rotometern. Die Tiere wurden in Plexiglas-Halbschalen (Durchmesser 40 cm) gesetzt und mechanisch mit einem Winkelschrittgeber verbunden. Die Bewegungen mit und entgegen dem Uhrzeigersinn wurden getrennt in 10-Minuten-Intervallen für 2 Stunden erfasst.

Die Ergebnisse dieser Versuche lassen sich wie folgt zusammenfassen:

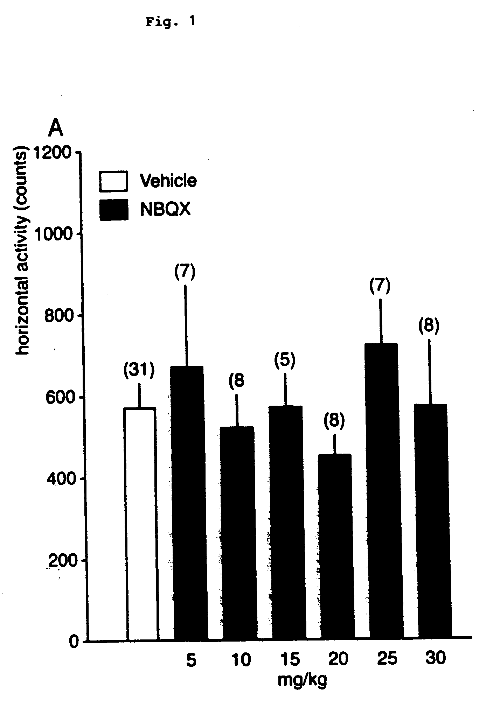

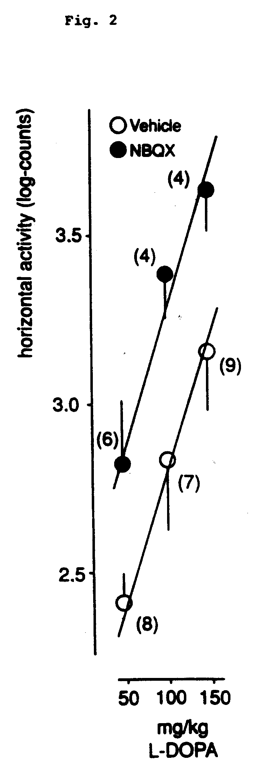

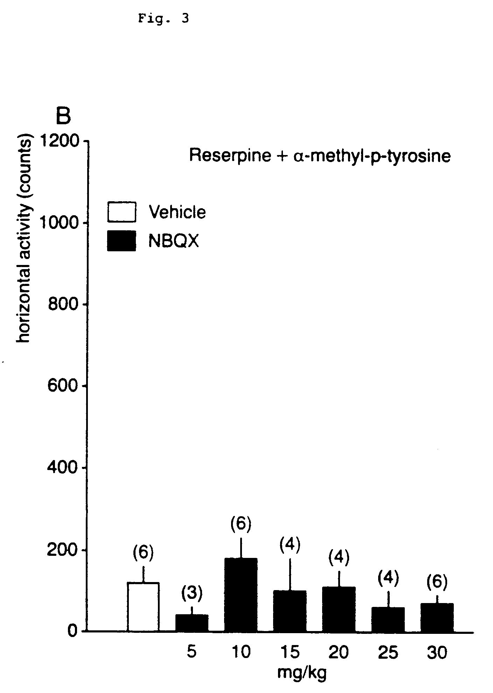

An normalen Ratten führt NBQX (10 - 30 mg/kg i.p.) zu keiner Steigerung der Lokomotion (Fig. 1). Die Entleerung der zentralen Monoaminspeicher durch Vorbehandlung mit Reserpin (5 mg/kg i.p. 24 Stunden vor dem Experiment) und α-Methyl-p-Tyrosin (250 mg/kg i.p. 4 Stunden vor dem Experiment) bewirkt eine massive Verminderung der normalen Lokomotion, die durch L-DOPA (50 - 150 mg/kg i.p.) (in Kombination mit Benserazid, 100 mg/kg i.p.) in dosisabhängiger Weise wieder aufgehoben werden kann (Fig. 2). NBQX (5 - 30 mg/kg i.p.) steigert die lokomotorische Aktivität reserpinisierter Ratten nicht (Fig, 3). Kombiniert man dagegen NBQX (5 mg/kg i,p,) mit L-DOPA (50 - 150 mg/kg i.p.), kommt es zu einer Linksverschiebung der L-DOPA Dosis-Wirkungskurve, so daß eine gleichbleibende Wirkung mit weniger als der Hälfte von L-Dopa erzielt werden kann (Fig. 2).

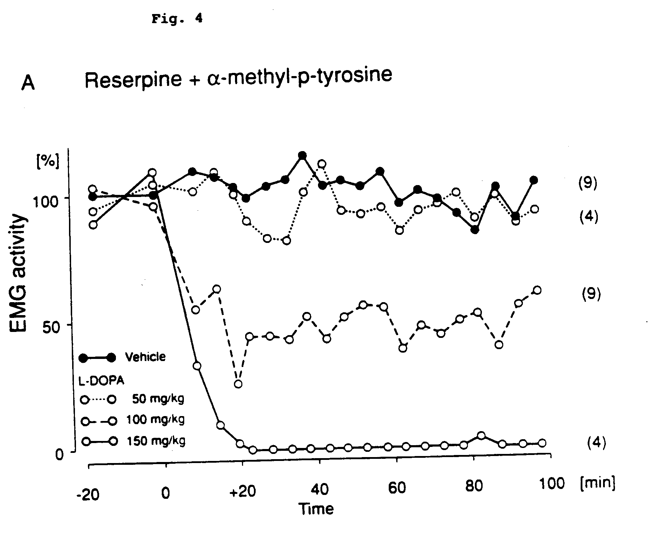

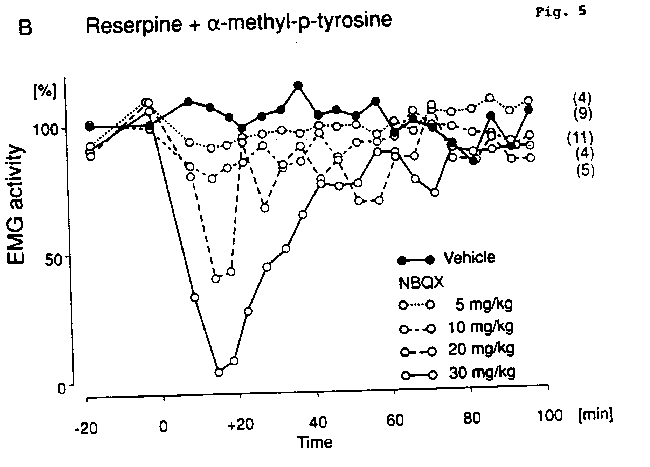

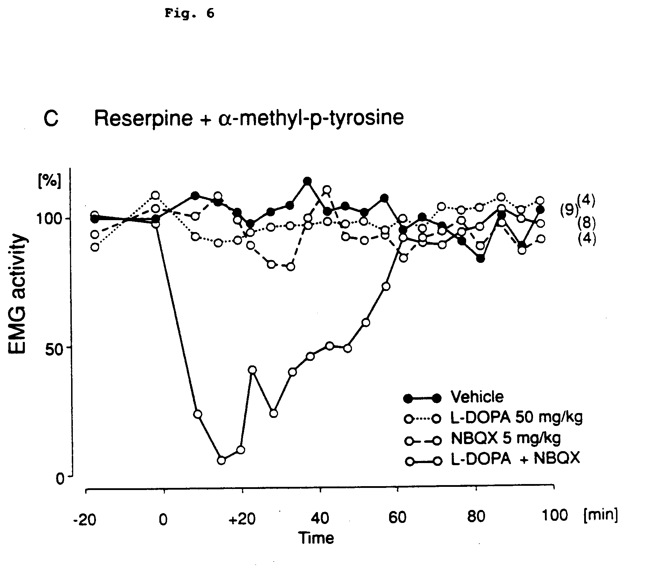

Die tonische EMG-Aktivität im GS reserpinisierter Ratten stellt ein quantitatives Maß der muskulären Rigidität dar. Sie kann durch L-DOPA (50 -150 mg/kg i.p.) in dosisabhängiger Weise unterdrückt werden (Fig. 4). NBQX (5 - 30 mg/kg i.p`) hat einen ähnlichen, jedoch kürzer anhaltenden Effekt (Fig. 5). Kombiniert man Schwellendosen von NBQX (5 mg/kg i.p.) und L-DOPA (50 mg/kg i.p,), die jeweils allein keinen Einfluß auf die EMG-Aktivität ausüben, kommt es zu einer vollständigen Unterdrückung der EMG-Aktivität (Fig. 6).

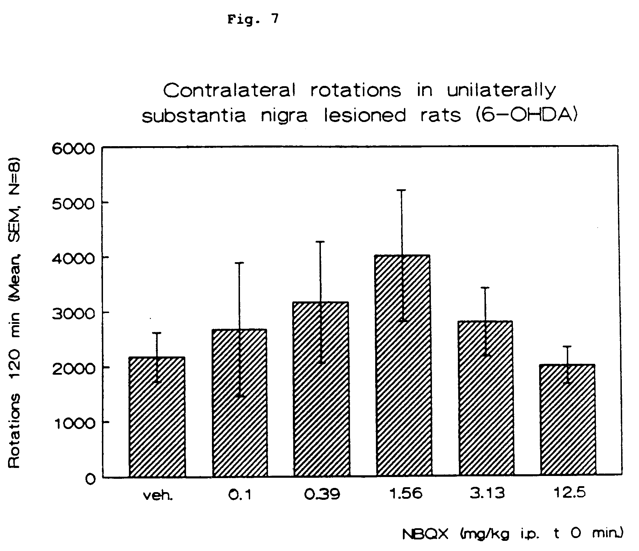

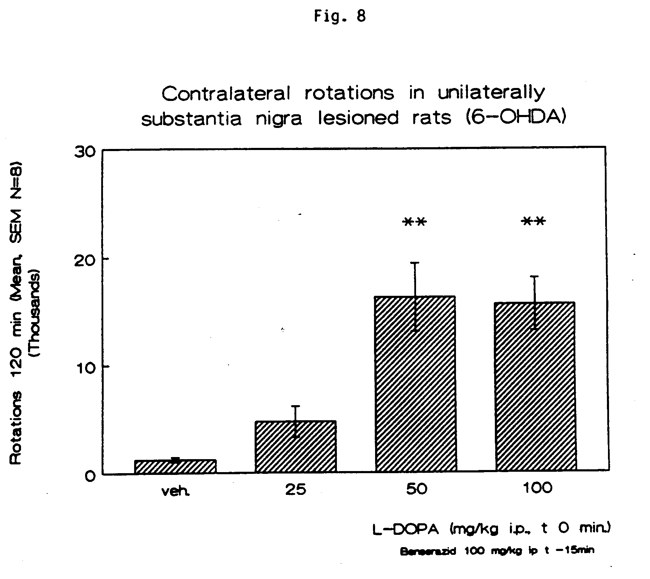

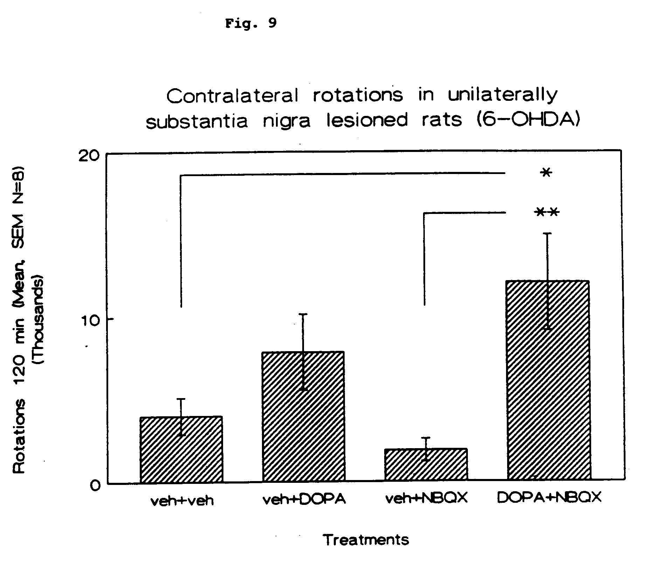

In Dosierungen von 0,78, 3,13 und 12,5 mg/kg i.p, sind durch NBQX weder kontralaterale noch ipsilaterale Rotationen auszulösen (Fig. 7). Dagegen führt L-DOPA (25, 50 und 100 mg/kg i.p.) nach Vorbehandlung mit Benserazid (100 mg/kg i.p. - 15 Minuten) zu einer dosisabhängigen Induktion kontralateraler Rotation (Fig. 8). Bei kombinierter Behandlung mit einer Schwellendosis von L-DOPA (25 mg/kg i.p.) und NBQX (12,5 mg/kg i.p. ), eine Dosis die per se inhibitorisch wirkt, ist eine signifikante Induktion kontralateraler Rotationen zu beobachten (Fig. 9).

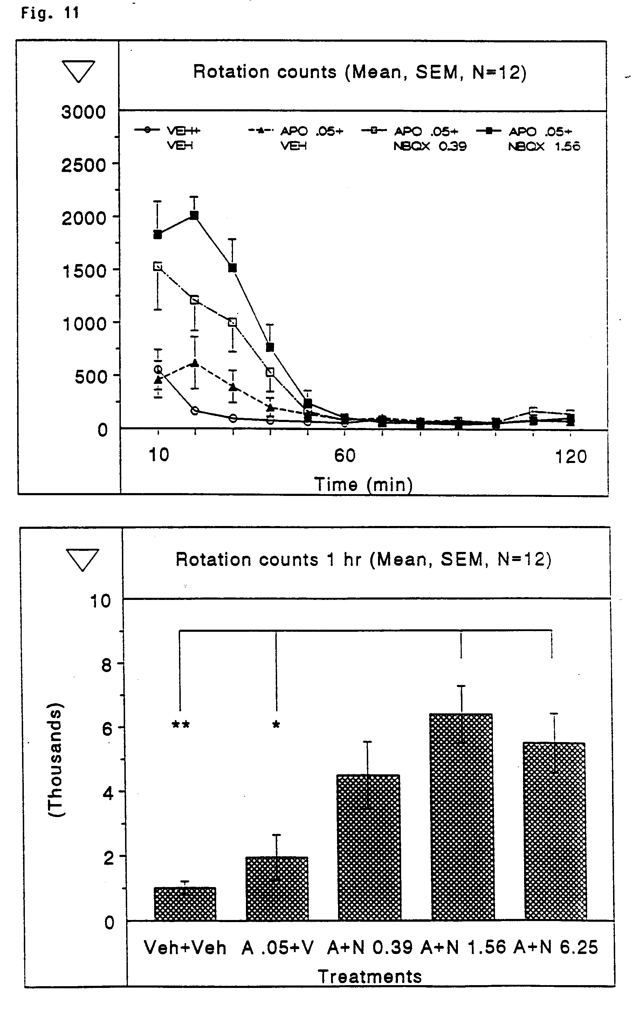

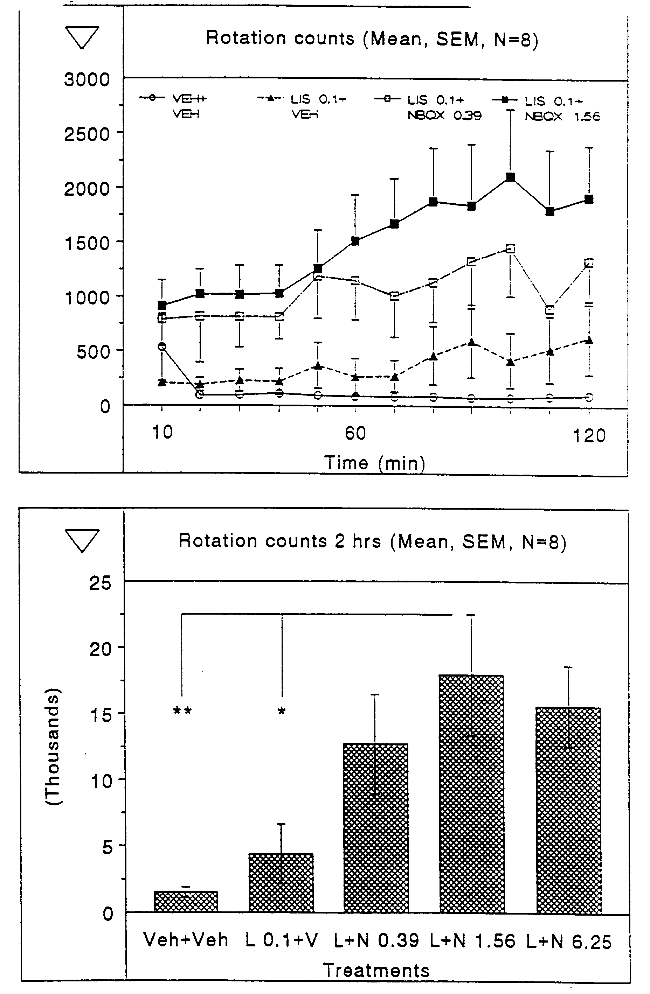

Im gleichen Modell wurde NBQX (0.39, 1.56, 6.25 mg/kg i.p.) mit den direkten Dopaminagonisten Apomorphin (0 .05 mg/kg i.p.) oder Lisurid (0.1 mg/kg i.p.) geprüft (Fig. 11 und Fig. 12). Auch hier führt die kombinierte Behandlung zu einem signifikantem Svnergismus (Varianzanalyse und Tukey Test, *p<0,05, **p<0.01).

In gleicher Weise wurde NBQX an Pinselohräffchen geprüft, bei denen durch das Neurotoxin MPTP ein Parkinsonsyndrom erzeugt worden war. Diese Affen zeigen wie der Parkinsonpatient eine starke Verminderung der Beweglichkeit. Für die hier beschriebenen Experimente wurden die Tiere in Käfige gesetzt, die mit Infrarotlichtschranken ausgerüstet wurden, Die Zahl der Lichtschrankenunterbrechungen wurde automatisch registriert und als Maß der Beweglichkeit über 2 Stunden gemessen,

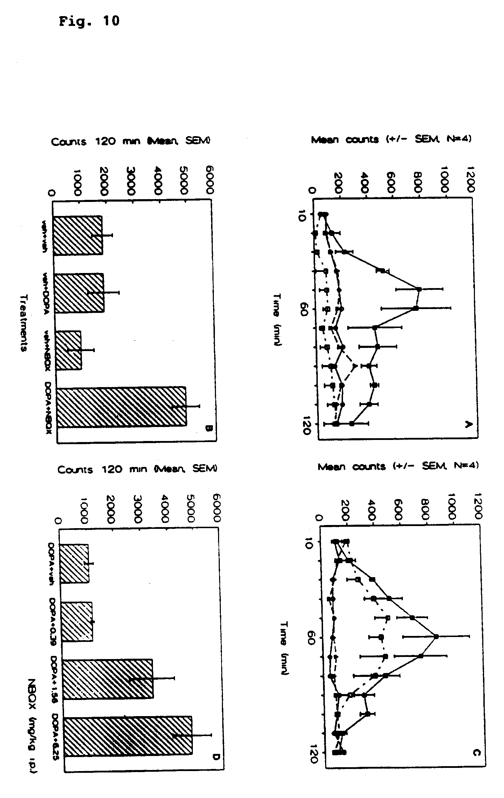

Gruppen von 4 Tieren wurden entweder mit Lösungsmittel, 20 mg/kg L-Dopa und 20 mg/kg Benserazid, 6,25 mg/kg NBQX oder einer Kombination von L-Dopa, Benserazid und NBQX in gleicher Dosierung behandelt. Die gewählte Dosis von L-Dopa hatte keinen Effekt, NBQX allein hemmte die Beweglichkeit, während die Kombinationsbehandlung zu einer statistisch signifikanten Stimulation der lokomotorischen Aktivität im Sinne eines Synergismus führte (Fig. 10 A + B). In einem weiteren Experiment wurde die gleiche Dosis von L-Dopa und Benserazid mit unterschiedlichen Dosen von NBQX (0,39; 1,56: 6,25 mg/kg i.p.) kombiniert. Hier konnte gezeigt werden, daß der Effekt von NBQX dosisabhängig ist (Fig. 10 C + D)

Diese Untersuchungen zeigen, daß Antagonisten der AMPA-Rezeptoren in Dosierungen, die allein ohne Wirkungen sind, nach systemischer Applikation die Wirkung von L-DDPA auf die Akinese und muskuläre Rigidität reserpinisierter Ratten verstärken. Auch die Wirkung von L-DOPA im 6-OHDA-Rotationsmodell und am MPTP-lädierten Primaten wird verstärkt.

Als Folge der Blockade der zentralen AMPA-Rezeptoren, also der Strukturen, die beim Morbus Parkinson eine erhöhte neuronale Aktivität aufweisen, eignen sich Verbindungen mit selektiver und nicht-selektiver Wirkung auf AMPA-Rezeptoren sowol zur symptomatischen Behandlung des Morbus Parkinson als auch zur Kombination mit herkömmlichen Antiparkinsonmitteln wie L-DOPA, L-DOPA in Kombination mit Benserazid und Dopaminagonisten wie beispielsweise Lisurid, Bromokriptin, Amantadin-Derivate, Memantin und seine Derivate und den in EP-A-351 352 beschriebenen Verbindungen.

Durch Kombination der erfindungsgemäßen Arzneimittel mit herkömmlichen Antiparkinsonmitteln wird die zu verabfolgende Dosis des herkömmlichen Arzneimittels verringert und deren Wirkung synergistisch gesteigert.

Auf Grund der Untersuchungsergebnisse üben die erfindungsgemäß verwendeten Quisqualat-Rezeptor-Antagonisten auch neuroprotektive Wirkung aus, die die Degeneration dopaminerger Neuronen verhindern, insbesondere gegenüber den möglichen neurotoxischen Wirkungen, die durch die herkömmlichen Antiparkinsonmittel hervorgerufen werden. Das Fehlen psychotomimetischer Nebenwirkungen ist ebenfalls vorteilhaft.

Die Erfindung umfaßt auch pharmazeutische Mittel, die die genannten Verbindungen enthalten, deren Herstellung sowie die Verwendung der erfindungsgemäßen Verbindungen zur Herstellung von Arzneimitteln, die zur Behandlung und Prophylaxe der vorstehend genannten Krankheiten eingesetzt werden, Die Arzneimittel werden nach an sich bekannten Verfahren hergestellt, indem man den Wirkstoff mit geeigneten Träger-, Hilfs- und/oder Zusatzstoffen in die Form eines pharmazeutischen Präparates bringt, das für die enterale oder parenterale Applikation geeignet ist. Die Applikation kann oral oder sublingual als Feststoff in Form von Kapseln oder Tabletten oder als Flüssigkeit in Form von Losungen, Suspensionen, Elixieren oder Emulsionen oder rektal in Form von Suppositorien oder in Form von gegebenenfalls auch subcutan anwendbaren Injektionslösungen erfolgen. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Öle, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen.

Als Trägersysteme können auch grenzflächennahe Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,001 -1 mg/kg Körpergewicht, wobei die Dosis als einmal zu verabreichende Einzeldosis oder unterteilt in 2 oder mehrere Tagesdosen gegeben werden kann.

In den erfindungsgemäßen Kombinationspräparaten mit synergistischer Wirkung können die Wirkstoffe in einer Formulierung oder auch in jeweils getrennten Formulierungen vorliegen, wobei die gesamte Dosis einmalig verabreicht oder in mehrere Dosen geteilt wird.

Die tägliche Dosis der Wirkstoffe in den Kombinationspräparaten beträgt für das herkömmliche Antiparkinsonmittel 2 mg bis 1500 mg und für den Quisqualat-Rezeptor-Antagonisten 1 mg bis 500 mg, besonders geeignet sind Dosen von 5 mg bis 100 mg.

## Patentansprüche

1. Mittel enthaltend (1) einen Quisqualat-Rezeptor-Antagonisten oder dessen physiologisch verträgliches Salz und (2) einen Dopamin-Agonisten.

2. Mittel nach Anspruch 1, worin die Komponente (1) ein Chinoxalindionderivat oder deren tautomere Form der Formel I ist 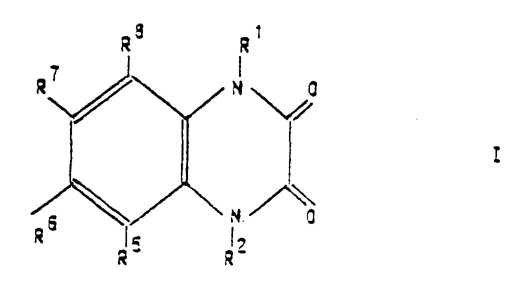 worin
R¹ und R² jeweils Wasserstoff oder gegebenenfalls mit Hydroxy, NH₂. Carboxy, Carbonsäureestern oder Carbonsäureamiden substituiertes C₁₋₁₂-Alkyl, C₃₋₈₋Cycloalkyl, C₄₋₈-Cycloalkylalkyl, C₆-₁₀-Aryl, C₇₋₁₁-Aralkyl C₂₋₇-Alkanoyloxy, Hydroxy, C₁₋₆-Alkoxy, C₆₋₁₀-Aryloxy, C₃₋₈-Cycloalkyloxy oder C₄₋₈-Cycloalkylalkyloxy darstellen und
R⁵, R⁶, R⁷ und R⁸ gleich oder verschieden sind und jeweils Wasserstoff NO₂, NH₂, Cyano, Halogen, CF₃, SO2NR'R', SO₂R' oder OR' bedeuten und R' Wasserstoff oder C₁₋₄-Alkyl ist oder
R⁵ und R⁶ oder R⁷ und R⁸ jeweils gemeinsam ankondensiertes Benzol, Pyridin, Pyrazol, Thiophen, Pyrazin, Triazol, Imidazol oder -(CH₂)₄- bedeuten, wobei der Benzol- oder Hetarylrest ein- bis dreifach gleich oder unterschiedlich substituiert sein kann mit NO₂, NH₂, Cyano, Halogen, CF₃, SO₂NR'R', SO₂R' oder OR' bedeuten, wobei R' die obige Bedeutung hat.

3. Mittel nach Anspruch 1 und 2, Worin die Komponente (1) 6-Nitro-7-sulfamoylbenzo[f]-quinoxalin-2,3-(1H, 4H)-dion (NBQX), 6,7-Dinitroquinoxalin 2,3-dion (DNQX) oder 6-Cyano-7-nitroquinoxalin-2,3-dion (CNQX) ist.

4. Mittel nach Anspruch 1, worin die Komponente (2), L-DOPA, L-DOPA in Kombination mit Benserazid, Lisurid, Bromokryptin, Apomorphin, ein Amantadin-Derivat, Memantin oder ein Memantin-Derivat ist.

5. Mittel nach Anspruch 1 bis 4 zur Prävention und Behandlung des Morbus Parkinson.

## Claims

1. Composition comprising (1) a quisqualate receptor antagonist or its physiologically tolerable salt and (2) a dopamine agonist.

2. Composition according to Claim 1, in which the component (1) is a quinoxalinedione derivative or its tautomeric form of the formula (1) 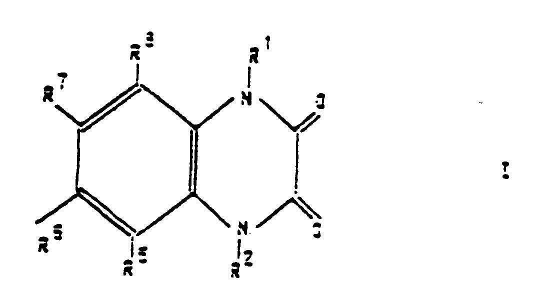 in which
R¹ and R² are each hydrogen or C₁₋₁₂-alkyl, C₃₋₈-cycloalkyl, C₄₋₈-cycloalkylalkyl, C₆-₁₀-aryl, C₇₋₁₁-aralkyl, C₂-₇-alkanoyloxy, hydroxyl, C₁-₆-alkoxy, C₆₋₁₀-aryloxy, C₃₋₈-cycloalkyloxy or C₄₋₈-cycloalkylalkyloxy, each of which is optionally substituted by hydroxyl, NH₂, carboxyl, carboxylic acid esters or carboxamides, and
R⁵, R⁶, R⁷ and R⁸ are identical or different and are each hydrogen, NO₂, NH₂, cyano, halogen, CF₃, SO₂NR'R', SO₂R' or OR' and R' is hydrogen or C₁₋₄-alkyl, or
R⁵ and R⁵ or R⁷ and R⁸ are each together fused benzene, pyridine, pyrazole, thiophene, pyrazine, triazole, imidazole or -(CH₂)₄-, where the benzene or hetaryl radical can be identically or differently mono- to trisubstituted by NO₂, NH₂, cyano, halogen, CF₃, SO₂NR'R', SO₂R' or OR', where R' has the above meaning.

3. Composition according to Claims 1 and 2, in which the component (1) is 6-nitro-7-sulphamoylbenzo[f]-quinoxaline-2,3-(1H,4H)-dione (NBQX), 6,7-dinitroquinoxaline-2,3-dione (DNQX) or 6-cyano-7-nitroquinoxaline-2,3-dione (CNQX).

4. Composition according to Claim 1, in which the component (2) is L-DOPA, L-DOPA in combination with benserazide, lisuride, bromocryptine, apomorphine, an amantadine derivative, memantine or a memantine derivative.

5. Composition according to Claims 1 to 4 for the prevention and treatment of Parkinson's disease.

## Revendications

1. Agent contenant (1) un antagoniste de récepteur du quisqualate ou un sel physiologiquement acceptable de celui-ci et (2) un agoniste de la dopamine.

2. Agent selon la revendication 1, dans lequel le composant (1) est un dérivé de quinoxalinedione ou sa forme tautomère de formule I 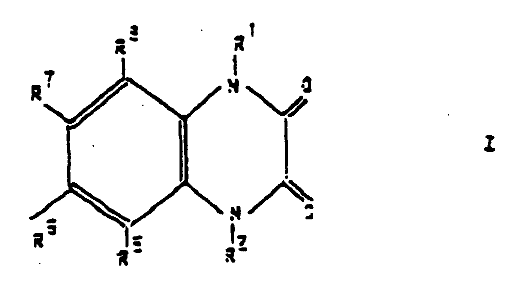 dans laquelle
R¹ et R² représentent chacun un atome d'hydrogène ou des radicaux alkyle en C₁₋₁₂ éventuellement substitués par des groupes hydroxyle, NH2, carboxyle, esters d'acides carboxyliques ou carboxamido, ou des radicaux cycloalkyle en C₃₋₈, cycloalkylalkyle en C₄₋₈, aryle en C₆₋₁₀, aralkyle en C₇₋₁₁, alcanoyloxy en C₂₋₇, hydroxyle, alcoxy en C₁₋₆, aryloxy en C₆₋₁₀, cycloalkyloxy en C₃₋₈ ou cycloalkylalkyloxy en C₄₋₈ et
R⁵, R⁶, R⁷ et R⁸ sont identiques ou différents et représentent chacun un atome d'hydrogène ou d'halogène, un groupe NO₂, NH₂, cyano, CF₃, SO₂NR'R', SO₂R' ou OR', et R' est un atome d'hydrogène ou un groupe alkyle en C₁₋₄, ou
R⁵ et R⁶ ou R⁷ et R⁸, respectivement, représentent ensemble un cycle benzénique les cycles pyridine, pyrazole, thiophène, pyrazine, triazole, imidazole soudé ou -(CH₂)₄, le radical benzénique ou hétéroaryle pouvant être 1 à 3 fois substitué de façon identique ou différente, par un ou des atomes d'halogène ou groupes NO₂, NH₂, cyano, CF₃, SO₂NR'R', SO₂R' ou OR', R' ayant la signification donnée plus haut.

3. Agent selon les revendications 1 et 2, dans lequel le composant (1) est la 6-nitro-7-sulfamoylbenzo[f]-quinoxaline-2,3-(1H,4H)dione (NBQX), la 6,7-dinitro-quinoxaline-2,3-dione (DNQX) ou la 6-cyano-7-nitro-quinoxaline-2,3-dione (CNQX).

4. Agent selon la revendication 1, dans lequel le composant (2) est la L-DOPA, la L-DOPA en association avec le bensérazide, le lisuride, la bromocryptine, l'apomorphine, un dérivé d'amantadine, la mémantine ou un dérivé de mémantine.

5. Agent selon les revendications 1 à 4, destiné à la prévention et au traitement de la maladie de Parkinson.
